(19) Europäisches Patentamt
European Patent Office
Office européen des brevets

(11) Veröffentlichungsnummer: **0 068 159**
**A1**

(12) **EUROPÄISCHE PATENTANMELDUNG**

(21) Anmeldenummer: **82104746.1**

(51) Int. Cl.³: **C 08 F 220/56**, A 61 L 15/00

(22) Anmeldetag: **29.05.82**

(30) Priorität: **22.06.81 DE 3124454**

(43) Veröffentlichungstag der Anmeldung: **05.01.83**
**Patentblatt 83/1**

(84) Benannte Vertragsstaaten: **DE FR GB IT**

(71) Anmelder: **CASSELLA Aktiengesellschaft, Hanauer Landstrasse 526, D-6000 Frankfurt am Main 61 (DE)**

(72) Erfinder: **Engelhardt, Friedrich, Dr., Hünfelder Strasse 20, D-6000 Frankfurt am Main 61 (DE)**
Erfinder: **Schmitz, Hermann, Dr., Marbachweg 313, D-6000 Frankfurt am Main 1 (DE)**
Erfinder: **Kleiner, Hans-Jerg, Dr., Altkönig Strasse 11a, D-6242 Kronberg 2 (DE)**
Erfinder: **Lask, Helmut, Taubenbergstrasse 7, D-6228 Eltville (DE)**
Erfinder: **Holst, Arno, Dr., Drususstrasse 3, D-6200 Wiesbaden (DE)**

(74) Vertreter: **Urbach, Hans-Georg, Dr. et al, Hanauer Landstrasse 526, D-6000 Frankfurt am Main 61 (DE)**

(54) **Wasserquellbare vernetzte Copolymerisate, ihre Herstellung und Verwendung.**

(57) Neue, in wäßrigen Systemen quellbare, vernetzte Copolymerisate, die in den Grundketten in statistischer Verteilung zu
0 bis 60 Gew.-% aus Resten der Formel I

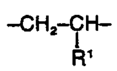
(I)

0 bis 40 Gew.-% aus Resten der Formel II

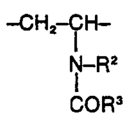
(II)

und 10 bis 95 Gew.-% aus Resten der Formel III

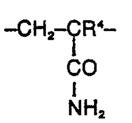
(III)

bestehen, wobei die Summe der Anteile der Reste der Formeln I und II 5 bis 90 Gew.-% beträgt, und die zusätzlich 0,001 bis 2 Gew.-%, bezogen auf das Gesamtgewicht der Grundkettenbestandteile vernetzende Brückenglieder der Formeln IV und/oder V

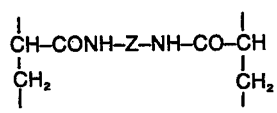
(IV)

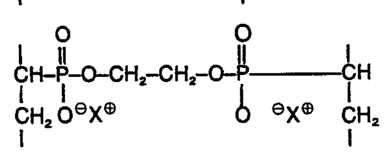
(V)

enthalten, worin $R^1$, $R^2$, $R^3$, $R^4$, $X^{\oplus}$ und Z, die im Anspruch 1 genannten Bedeutungen haben, ihre Herstellung und ihre Verwendung als Absorptionsmittel mit besonders hohem Absorptionsvermögen für wäßrige Flüssigkeiten, insbesondere für physiologische Flüssigkeiten.

EP 0 068 159 A1

0068159

Ref. 3219
Dr.Va/lro

Wasserquellbare vernetzte Copolymerisate, ihre Herstellung und Verwendung

Die vorliegende Erfindung betrifft neue, in wäßrigen Systemen quellbare, vernetzte Copolymerisate, die in den Grundketten in statistischer Verteilung zu

0 bis 60 Gew.% aus Resten der Formel I

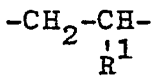 (I)

0 bis 40 Gew.% aus Resten der Formel II

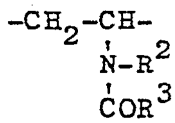 (II)

und 10 bis 95 Gew.% aus Resten der Formel III

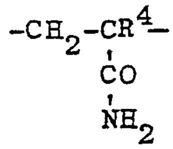 (III)

bestehen, wobei die Summe der Anteile der Reste der Formeln I und II 5 bis 90 Gew.% beträgt, und die zusätzlich 0,001 bis 2 Gew.%, bezogen auf das Gesamtgewicht der Grundkettenbestandteile vernetzende Brückenglieder der Formeln IV und/oder V

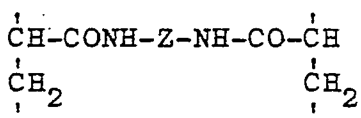 (IV)

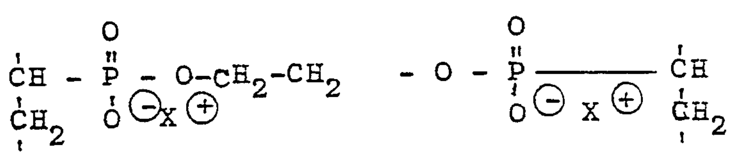 (V)

enthalten, worin $R^1$, $R^2$, $R^3$, $R^4$, $X^{\oplus}$, und Z die im Anspruch 1 genannten Bedeutungen haben, ihre Herstellung und ihre Verwendung als Absorptionsmittel mit besonders hohem Absorptionsvermögen für wäßrige Flüssigkeiten, insbesondere für physiologische Flüssigkeiten.

Die Herstellung wasserlöslicher unvernetzter Polymerisate, welche Sulfonsäuregruppen im Makromolekül eingebaut enthalten, ist bereits in zahlreichen Patenten sowie in der Fachliteratur ausführlich beschrieben worden. So ist z.B. die Synthese von Copolymeren der Vinylsulfonsäure mit Acrylamid und Vinylpyrrolidon im J. Polymer Sci., 38, 147 (1959) veröffentlicht worden. Im DBP 1 101 760 ist ein Verfahren zur Herstellung wasserlöslicher, unvernetzter Copolymerisate aus Vinylsulfonsäure und Acrylnitril bzw. Methacrylnitril gegebenenfalls im Gemisch mit weiteren ethylenisch ungesättigten Verbindungen beschrieben worden. Unvernetzte Copolymerisate aus Vinyl- bzw. Alkenylsulfonaten mit Acrylamid und Vinylamiden sind z.B. in der DAS 2 444 108 beschrieben worden. Unvernetzte wasserlösliche Copolymerisate, welche 2-Acrylamido-2-methyl-propansulfonsäure, im folgenden mit AIBS abgekürzt, als Comonomeres enthalten, sind in USP 3 953 342, 3 768 565, DOS 2 502 012, 2 547 773, USP 3 907 927, 3 926 718, 3 948 783 beschrieben. So lassen sich z.B. gemäß den Angaben des USP 3 929 741, Beispiel 10, Copolymere aus Vinylpyrrolidon und AIBS herstellen. Wasserlösliche, unvernetzte Copolymere aus AIBS bzw. Vinylsulfonsäure, Vinylacylaminen der Formel II sowie Acrylamid sind auch Gegenstand der älteren Patentanmeldungen P 29 31 897.6 und P 30 27 422.7.

Es ist auch bekannt, vernetzte Polymere auf der Basis von Polyacrylsäure und/oder deren K, Na bzw. Ammoniumsalze als flüssigkeitsabsorbierendes Material in Hygieneartikeln wie z.B. Windeln, Binden und Tampons zu verwenden.

So werden in der DE AS 1 642 072 (Anmelder Johnson + Johnson) entsprechende Hydrokolloide und deren Anwendung beschrieben. Ebenfalls beinhaltet die DE OS 161 79 98 (Anmelder Dow Chemical Co.) vernetzte Gelpolymerisate, unter anderem auf der Basis von N-Vinylpyrrolidon bzw. Acrylamid.

Aus der DE OS 2 706 135 (Anmelder Chemische Fabrik Stockhausen) sind Verdickungsmittel für durch künstliche Ausgänge ausgeschiedenen Darminhalt und/oder Harn bekannt, gekennzeichnet durch einen Gehalt an wenigstens einem vernetzten, in

Wasser nur noch quellbaren Homopolymeren auf der Basis u.a.von Acrylsäure, Acrylamid oder Copolymeren aus wenigstens zwei der Acrylmonomeren, Acrylsäure, Methacrylsäure, Acrylamid, Methacrylamid und Acrylnitril untereinander, ferner aus wenigstens einem dieser Acrylmonomeren und Vinylpyrrolidon.

Flüssigkeitsabsorbierende Substanzen, insbesondere solche, die in Hygieneartikeln eingesetzt werden, sind umso vorteilhafter in der Anwendung, je mehr Flüssigkeit sie pro Gewichtseinheit zu binden vermögen.
Es wurde nun gefunden, daß neue wasserquellbare vernetzte Copolymerisate, die in den Grundketten in statistischer Verteilung zu

0 bis 60 Gew.% aus Resten der Formel I

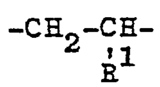
(I)

0 bis 40 Gew.% aus Resten der Formel II

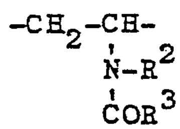
(II)

und 10 bis 95 Gew.% aus Resten der Formel III

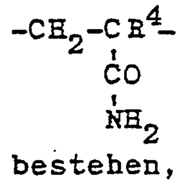
(III)

bestehen, wobei die Summe der Anteile der Reste der Formeln I und II 5 bis 90 Gew.% beträgt, und die zusätzlich 0,001 bis 2 Gew.%, bezogen auf das Gesamtgewicht der Grundkettenbestandteile vernetzende Brückenglieder der Formeln IV und/oder V

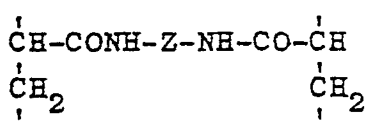
(IV)

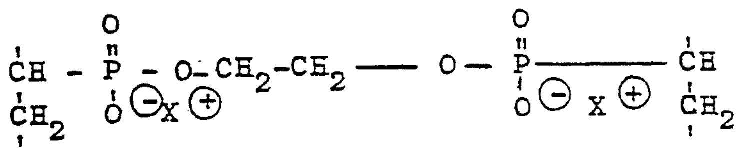
(V)

enthalten, worin

$R^1$ einen der Reste der Formeln

$-CONH-C(CH_3)_2-CH_2-SO_3^{\ominus} X^{\oplus}$,

$-CO-NH-C(CH_3)_2-CH_2-PO_3^{\ominus\ominus} X_2^{\oplus}$

$-SO_3^{\ominus} X^{\oplus}$, $-PO_3^{\ominus\ominus} X_2^{\oplus}$ oder $-\overset{O}{\overset{\|}{P}}(OR^5)_2$ bedeutet,

$R^2$ und $R^3$ gleich oder verschieden sind und für Wasserstoff, Methyl, Ethyl oder gemeinsam für Trimethylen stehen, $R^4$ Wasserstoff oder Methyl, $R^5$ Alkyl mit 1 bis 4 C-Atomen, $X^{\oplus}$ ein Kation und Z eine der Gruppen $-CH_2-$; $-CH_2CH_2-$;

$$-\underset{}{\overset{COO^{\ominus}X^{\oplus}}{\overset{|}{CH}}}-$$

oder $-CH_2-O-CH_2-$ ist, sowie deren Partialhydrolysate, in denen ein Anteil von bis zu 60 % der ursprünglich vorhandenen Gruppen der Formel III zu Gruppen der Formel VI

$$-CH_2-\underset{COO^{\ominus} X^{\oplus}}{\underset{|}{CH}}- \qquad (VI)$$

hydrolysiert wurden, wobei die Produkte insgesamt 10 bis 90 Gew.% anionische Reste aufweisende Gruppen der Formeln I und VI enthalten, für den Hygienesektor ebenfalls mit Erfolg einsetzbar sind und daß sie gegenüber bekannten hydrophilen synthetischen Polymeren auf Acrylatbasis überraschenderweise ein höheres Aufnahme- und Rückhaltevermögen von Elektrolytlösungen besitzen.

Unter den Grundketten der erfindungsgemäßen Copolymerisate sind die Haupt-Polymerenstränge zu verstehen, die sich im wesentlichen aus den Bauelementen der Formeln I, II und III zusammensetzen.

Die Struktur der erfindungsgemäßen wasserquellbaren, vernetzten Copolymerisate ist somit durch die folgende Strukturformel darstellbar :

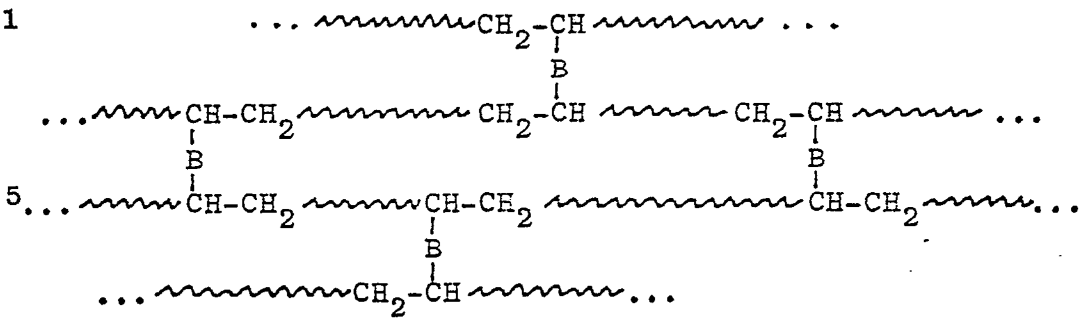

worin die Zickzacklinien die Hauptpolymerenstränge, d.h. die Grundketten, und B Brückenglieder der Formeln

$$-CO-NH-Z-NH-CO- \quad \text{oder} \quad -\overset{O}{\overset{\|}{P}}(O^{\ominus}X^{\oplus})-O-CH_2CH_2- \quad -O-\overset{O}{\overset{\|}{P}}(O^{\ominus}X^{\oplus})-$$

bedeuten.

Das Kation $X^{\oplus}$ kann ein Proton sein oder sich von jeder wasserlöslichen bekannten Base ableiten, deren Stärke ausreicht, die Sulfo- und Carboxylgruppen der erfindungsgemäßen Copolymere zu neutralisieren und die die Wasserquellbarkeit der Copolymeren nicht beeinträchtigt. Die Auswahl kann somit in einfacher bekannter Weise erfolgen.

Zweckmäßigerweise bedeutet jedoch $X^{\oplus}$ ein Proton oder ein Alkalikation, insbesondere ein Natrium- oder Kaliumkation, oder ein Kation der Formel $\overset{\oplus}{H}NR^6_3$, wobei die drei Reste $R^6$ gleich oder verschieden sind und für Wasserstoff, Alkyl mit 1 bis 4 C-Atomen, Hydroxyethyl oder Hydroxypropyl stehen.

Bevorzugte erfindungsgemäße Copolymerisate bestehen in den Grundketten in statistischer Verteilung zu 0 bis 50 Gew.% aus Resten der Formel I, 0 bis 30 Gew.% aus Resten der Formel II, und zu 20 bis 95 Gew.% aus Resten der Formel III bzw. deren Hydrolyseprodukte. Bevorzugt sind auch solche erfindungsgemäßen Copolymerisate, in denen $R^4$ Wasserstoff ist. Vorzugsweise enthalten die erfindungsgemäßen vernetzten Copolymerisate, bezogen auf das Gesamtgewicht der Grundkettenbestandteile, 0,02 bis 0,5 Gew.% Reste der Formeln IV und V.

Besonders bevorzugte erfindungsgemäße Copolymerisate enthalten Reste der Formel I, in denen $R^1$ den Rest

$-CO-NH-C(CH_3)_2-CH_2-SO_3^{\ominus}\ Na^{\oplus}$ und/oder

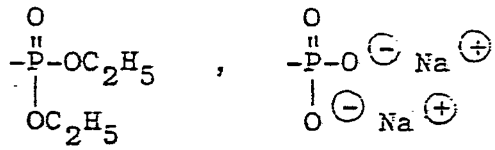

bedeutet und/oder Reste der Formel II mit $R^2 = R^3 = H$ oder $R^2 = R^3 = CH_3$ oder $R^2 = H$ , $R^3 = CH^3$.

In den durch Partialhydrolyse der erfindungsgemäßen Copolymerisate erhältlichen Derivaten ist ein Anteil von bis zu 60%, vorzugsweise 10 - 40%, der ursprünglich vorhandenen Reste der Formel III durch Reste der Formel VI

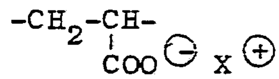 VI

ersetzt, wobei $X^{\oplus}$ die oben genannten Bedeutungen hat. Zweckmäßigerweise wird im Rahmen dieses Bereichs der Hydrolysegrad umso höher gewählt, je geringer der Sulfonsäure- bzw. Phosphonsäure— Gehalt im Copolymeren ist und umgekehrt.

Bevorzugte Partialhydrolysate der erfindungsgemäßen Copolymerisate enthalten insgesamt 20 bis 80 Gew.% von Gruppen der Formeln I und VI.

Selbstverständlich können in ein und demselben Copolymerisat auch die Reste $R^1$ bis $R^6$, Z und $X^{\oplus}$ im Rahmen ihrer Definitionen verschiedene Bedeutungen haben. So ist es beispielsweise möglich, daß darin verschiedene der angegebenen Brückenglieder vorkommen und daß erfindungsgemäße Copolymerisate sowohl AMPS_Bausteine als auch Vinylsulfonsäurebausteine aufweisen oder z.B. sowohl Vinylpyrrolidon als auch ein nicht ringgeschlossenes N-Vinyl-N-alkyl-acylamid wie z.B. N-Vinyl-N-methyl-acetamid oder neben Acrylamid auch Methacrylamid einpolymerisiert enthalten.

Das sehr gute Absorptionsvermögen für physiologische und elektrolythaltige Flüssigkeiten der erfindungsgemäßen Copolymerisate und ihrer Partialhydrolysate wird in vielen Fällen durch Anwesenheit von Borat-Anionen noch weiter erhöht.

Diese vorteilhaften, Borat-Anionen enthaltenden erfindungsgemäßen Copolymerisate und deren Partialhydrolysate weisen, berechnet als $H_3BO_3$, 2,5 bis 35 Gew.%, vorzugsweise 5 bis 25 Gew.% Borat-Anionen auf, bezogen auf das Gewicht des unverseiften Polymeren.

Außer auf den günstigen Eigenschaften der im Copolymer enthaltenen funktionellen Gruppen beruht die gegenüber den bisher bekannten Copolymeren höhere Quellfähigkeit in Salzlösungen und physiologischen Flüssigkeiten vermutlich auf einer zusätzlichen Interaktion der Borat-Anionen mit dem polymeren Netzwerk.

Die erfindungsgemäßen wasserquellbaren vernetzten Copolymerisate werden erhalten, wenn man
0 bis 60 Gewichtsteile einer Vinylverbindung der Formel Ia

$$CH_2{=}CH{-}R^1 \qquad \text{(Ia)}$$

in Wasser löst, sofern sie Sulfo- oder Phosphonsäuregruppen enthalten, neutralisiert, danach
0 bis 40 Gewichtsteile einer Vinylverbindung der Formel IIa

$$CH_2{=}CH{-}\overset{R^2}{\overset{|}{N}}{-}CO{-}R^3 \qquad \text{(IIa)}$$

und 10 bis 95 Gewichtsteilen Acrylamid und / oder Methacrylamid zusetzt, wobei die Gesamtmenge der Vinylverbindungen der Formeln Ia und IIa 5 bis 95 Gewichtsteile und die Gesamtmenge von Acrylamid und der Vinylverbindungen der Formeln Ia und IIa 100 Gewichtsteile beträgt, zusätzlich m Gewichtsteile von Bis-vinylverbindungen der Formeln IVa und Va

$$CH_2{=}CH{-}CONH{-}Z{-}NH{-}CO{-}CH{=}CH_2 \qquad \text{(IVa)}$$

$$CH_2{=}CH{-}\overset{O}{\overset{\|}{\underset{O^{\ominus}X^{\oplus}}{\underset{|}{P}}}}{-}O{-}CH_2{-}CH_2{-}O{-}\overset{O}{\overset{\|}{\underset{O^{\ominus}X^{\oplus}}{\underset{|}{P}}}}{-}CH{=}CH_2 \qquad \text{(Va)}$$

zufügt, wobei m eine Zahl von 0,001 bis 2 ist und $R^1$, $R^2$, $R^3$, $X^{\oplus}$, und Z die obengenannten Bedeutungen haben, und daß man die Copolymerisation in an sich bekannter Weise einleitet und bei 0 bis 130°C, vorzugsweise 10 - 100°C, durchführt.

Will man während der Copolymerisation bereits eine Teilhydrolyse der Polymerisate durchführen, so wird der Monomerenmischung zusätzlich ein alkalisches Verseifungsmittel zugefügt. Als Verseifungsmittel kann prinzipiell jede wasserlösliche Base eingesetzt werden, deren Stärke für eine Reaktion mit den hydrolysierbaren Gruppen ausreicht, wie z.B. Alkali- und Erdalkalihydroxyde, Alkali- oder Ammoniumsalze schwacher Säuren, die durch Hydrolyse $OH^{\ominus}$-Ionen generieren, Ammoniak oder stark basische aliphatische Amine. Zweckmäßigerweise werden als Verseifungsmittel Natrium- oder Kaliumhydroxyd, Ammoniak , Natrium- oder Kaliumcarbonat, -hydrogencarbonat, -phosphat, -borat oder -acetat eingesetzt. Soll die Teilhydrolyse erst nach Polymerisation erfolgen, so wird das Verseifungsmittel mit dem Polymerisat vermischt. Die Verseifung erfolgt daher - je nach Verfahrensweise - bereits während der normalerweise exotherm verlaufenden Polymerisation oder durch weitere Wärmezufuhr im Anschluß an die Polymerisation. Auch eine Kombination beider Verfahrensweisen ist möglich.

Die in vielen Fällen vorteilhaften, Borat-Anionen enthaltenden Produkte können auf einfache Weise erhalten werden, indem Natrium- oder Kaliumsalze der Borsäure (Ortho-, Meta- oder Polyborate bzw. Borsäure zusammen mit NaOH oder KOH) als Verseifungsmittel eingesetzt werden.

Es ist nicht unbedingt erforderlich, wenn auch in der Regel üblich, eine zur Menge der zu verseifenden Gruppen äquivalente Menge des Verseifungsmittels einzusetzen. Einerseits wird bei der Verseifung $NH_3$ frei, welches seinerseits die Verseifung weiterer Amidgruppen katalysieren kann, andererseits reicht der pH-Wert des aus Carboxylgruppen und Carboxylatgruppen des Copolymeren gebildeten Puffersystems ebenfalls zur weiteren Verseifung von Amidgruppierungen aus.

Bevorzugte erfindungsgemäße Copolymerisate werden erhalten, wenn man unter den oben angegebenen Voraussetzungen 0 bis 50 Gew.Teile Vinylverbindungen der Formel Ia, 0 bis 30 Gew. Teile Vinylverbindungen der Formel IIa und 10 bis 95 Gewichts-

1 Acrylamid einsetzt.

Bevorzugte erfindungsgemäße Produkte werden auch erhalten, wenn man 0,02 bis 0,5 Gew.Teile von Bis-vinylverbindungen der Formeln IVa und/oder Va als Vernetzer einsetzt.

Besonders vorteilhafte teilhydrolisierte erfindungsgemäße Copolymerisate werden erhalten, wenn soviel anionische Gruppen enthaltende Vinylverbindungen der Formel Ia eingesetzt und die Hydrolyse so weit geführt wird, daß die Produkte insgesamt 20 bis 80 Gew.% anionische Reste aufweisende Gruppen der Formeln I und VI enthalten.

Als Verbindungen der Formel Ia kommen in Betracht Vinylsulfonsäure, Vinylphosphonsäure, Vinylphosphonsäure-dimethyl-, -diethyl-, -dipropyl- und -dibutylester, 2-Acrylamido-2-methyl-propansulfonsäure-(1) und 2-Acrylamido-2-methyl-propanphosphonsäure-(1), und als Verbindungen der Formel IIa N-Vinylformamid, N-Vinylacetamid, N-Vinyl-propionamid, N-Vinyl-N-methylformamid, N-Vinyl-N-methylacetamid, N-Vinyl-N-methylpropionamid, N-Vinyl-N-ethyl-formamid, N-Vinyl-N-ethylacetamid, N-Vinyl-N-ethyl-propionamid, N-Vinylpyrrolidon.

Als vernetzend wirkende Monomerenbausteine können bei der Herstellung der erfindungsgemäßen Copolymeren Bis-acrylamidomethan, Bis-acrylamidoessigsäure, 1,1'-Bis-acrylamidodimethylether und Bis-vinyl-phosphonsäureglycolester, letzterer vorzugsweise in Form des bei seiner Herstellung anfallenden Rohprodukts, eingesetzt werden. Für den Aufbau der Grundketten werden neben Acrylamid und ggf. Methacrylamid vorzugsweise 2-Acrylamido-2-methylpropansulfonsäure, Vinylphosphonsäure, Vinylphosphonsäurediethylester, Vinylformamid, Vinylacetamid, Vinylmethylacetamid und Vinylpyrrolidon, als Vernetzer-Komponenten vorzugsweise Methylen-bis-acrylamid oder Bis-vinylphosphonsäure-glykolester bzw. dessen Rohprodukt eingesetzt.

Die Herstellung der erfindungsgemäßen Copolymerisate kann nach allen im Prinzip bekannten Polymerisationsverfahren wie Fällungs-, Emulsions-, Suspensions-, Lösungs- oder Gelpolymerisation erfolgen.

Eine mögliche Ausführungsform des Verfahrens zur Herstellung der erfindungsgemäßen wasserquellbaren, vernetzten Copolymerisate besteht darin, daß nach der Methode der Fällungspolymerisation in einem organischen Lösungsmittel, insbesondere in einem wasserlöslichen Alkanol, also in einem $C_1$-bis $C_4$-Alkohol wie Methanol, Ethanol, Isopropanol, n-, sec- und iso-Butanol, vorzugsweise aber in tert.-Butanol, gearbeitet wird.

Der Wassergehalt der als Lösungsmittel eingesetzten niederen Alkanole sollte 6 Gewichts% nicht überschreiten, da sonst eine Klumpenbildung bei der Polymerisation auftreten kann. Vorzugsweise wird bei einem Wassergehalt von 0 - 3% gearbeitet.

Die Menge des einzusetzenden Lösungsmittels richtet sich bis zu einem gewissen Grad nach der Art der eingesetzten Comonomeren.

In der Regel werden pro 100 g Gesamtmonomere 200 bis 1000 g des Lösungsmittels eingesetzt.

Wie üblich kann die Polymerisation in einer Schutzgasatmosphäre, vorzugsweise unter Stickstoff, ausgeführt werden. Die Polymerisationstemperatur liegt zwischen 20 und 120°C, vorzugsweise 40 bis 80°C.

Bei der Durchführung der Copolymerisation in den genannten organischen Lösungsmitteln, vorzugsweise in tert.-Butanol, unter den Bedingungen der Fällungspolymerisation, fällt das Polymerisat direkt in fester Form an und kann durch Abdestillieren des Lösungsmittels oder Absaugen und Trocknen isoliert werden. Das so hergestellte vernetzte Copolymerisat kann auch vor der Isolierung durch Zusatz von oben angegebenen Verseifungsmitteln, vorzugsweise von NaOH oder KOH, in Suspension bei Raumtemperatur oder erhöhter Temperatur teilhydrolisiert werden.

Bei der Verwendung von Wasser als Reaktionsmedium für die

Durchführung der erfindungsgemäßen Herstellung verläuft die Polymerisation zwar unter den Bedingungen der Lösungspolymerisation, jedoch wird ein wasserunlösliches, aber stark gequollenes erfindungsgemäßes Copolymerisat erhalten, das durch Abdestillieren des Wassers oder Ausfällung durch Zusatz eines wassermischbaren organischen Lösungsmittels wie Methanol, Ethanol, Aceton oder dgl. isoliert werden kann.

Besonders wirksame erfindungsgemäße Copolymerisate werden erhalten, wenn man die Polymerisation in Wasser nach dem Verfahren der sogenannten Gelpolymerisation durchführt. Dabei werden 15-50%ige wäßrige Lösungen der Comonomeren mit bekannten geeigneten Katalysatorsystemen und ggf. mit einem der genannten Verseifungsmittel ohne mechanische Durchmischung unter Ausnützung des sog. Trommsdorff-Norrish-Effektes (Bios Final Rep. 363 22; Makromol. Chem. 1 , 169 (1947)) polymerisiert. Die Polymerisationsreaktion kann im Temperaturbereich zwischen $0^{o}C$ und $130^{o}C$, vorzugsweise zwischen $10^{o}C$ und $100^{o}C$, sowohl bei Normaldruck als auch unter erhöhtem Druck durchgeführt werden. Wie üblich kann die Polymerisation auch in einer Schutzgasatmosphäre, vorzugsweise unter Stickstoff ,ausgeführt werden. Durch mehrstündiges Nachheizen der Polymerisatgele im Temperaturbereich von 50-$130^{o}C$, vorzugsweise 70-$100^{o}C$, können die Qualitätseigenschaften der Polymerisate noch verbessert werden. Die auf diesem Wege hergestellten, in Form wäßriger Gallerten vorliegenden erfindungsgemäßen Copolymerisate können nach Entfernung des Wassers durch bekannte Trocknungsprozesse in fester Form erhalten und mit allgemein bekannten Mahlaggregaten in Pulverform überführt werden.

Zur Auslösung der erfindungsgemäßen Polymerisationen können energiereiche elektromagnetische Strahlen oder die üblichen chemischen Polymerisationsinitiatoren herangezogen werden, z. B. organische Peroxide, wie Benzoylperoxid, tert. Butyl-hydroperoxid, Methyl-ethyl-keton-peroxid, Cumol-hydroperoxid, Azoverbindungen wie Azo-di-iso-butyro-nitril sowie anorganische Peroxiverbindungen wie $(NH_4)_2S_2O_8$ oder $K_2S_2O_8$ oder $H_2O_2$ gegebenenfalls in Kombination mit Reduktionsmitteln wie Natriumhydrogensulfit, oder Redoxsysteme, welche als reduzierende

Komponente eine aliphatische und aromatische Sulfinsäure, wie Benzolsulfinsäure und Toluolsulfinsäure oder Derivate dieser Säuren enthalten, wie z.B. Mannichaddukte aus Sulfinsäure, Aldehyden und Aminoverbindungen, wie sie in der deutschen Patentschrift 1 301 566 beschrieben sind. Pro 100 g Gesamtmonomeren werden in der Regel 0,03 bis 2 g des Polymerisationsinitiators eingesetzt.

Den als Vernetzer erfindungsgemäß eingesetzten Bis-vinylphosphonsäure-glycolester bzw. dessen Rohprodukt kann man auf einfache und wirtschaftliche Weise herstellen, indem man 2-Chlorethylestergruppen enthaltende 2-Chlorethanphosphonsäurederivate, insbesondere 2-Chlorethanphosphonsäure-bis-2-chlorethylester, die man bekanntlich durch Arbusow Umlagerung von Tris-chlorethylphosphit erhält, auf 150 bis 230°C, vorzugsweise 170 bis 215°C, erhitzt, wobei 1,2-Dichlorethan abgespalten wird. Die Abspaltung kann gegebenenfalls in Gegenwart saurer oder basischer Katalysatoren durchgeführt werden. Weiterhin kommen Salze der unterphosphorigen Säure als Katalysatoren in Frage.

Katalysatoren, die für die Abspaltung des 1,2-Dichlorethans besonders geeignet sind, sind beispielsweise Salze der unterphosphorigen Säure und Alkalikarbonate, wie Soda oder Pottasche.

Die Herstellung wird im allgemeinen so durchgeführt, daß die Ausgangsprodukte in Gegenwart von 0,1 bis 5 Gew.% der genannten Katalysatoren auf die Reaktionstemperatur erhitzt werden. Dann beginnt die Abspaltung des 1,2-Dichlorethans, in der Regel begleitet durch gleichzeitige Abspaltung geringerer Mengen Chlorwasserstoff. Das sich abspaltende 1,2-Dichlorethan destilliert in der Regel unter Normaldruck ab, gegebenenfalls unter Zuhilfenahme eines Inertgasstroms. Als Inertgas kommt insbesondere Stickstoff in Frage. Die Reaktionstemperaturen liegen bei 150 bis 230°C, vorzugsweise 170 bis 215°C. Höhere Temperaturen sind möglich, bringen aber keinen Vorteil. Die Reaktionsprodukte bestehen aus Gemischen von Vinylphosphonsäurederivaten, wobei

die Art und die Menge der einzelnen Bestandteile nicht bekannt sind. Im idealisierten Fall erhält man, ausgehend von 2-Chlorethanphosphonsäure-bis-2-chlorethylester, ein Gemisch der Verbindungen der Formeln

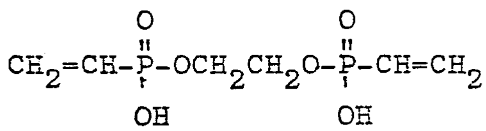 , 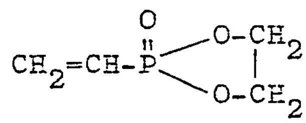 und

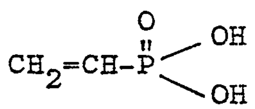 .

Diese Gemische von verschiedenen Vinylphosphonsäurederivaten können direkt ohne Isolierung einzelner Verbindungen, gegebenenfalls in Form der Salze mit dem Kation $X^{\oplus}$, als Vernetzer bei der Herstellung der erfindungsgemäßen Copolymerisate eingesetzt werden.

In den folgenden Ausführungsbeispielen und den Beispielen der Tabelle 1, die die Herstellung der erfindungsgemäß einzusetzenden wasserquellbaren, neuartigen Copolymeren zeigen, sind die folgenden Abkürzungen verwendet worden:

| | | |
|---|---|---|
| AM | : | Acrylamid |
| MAM | : | Methacrylamid |
| ViMA | : | N-Vinyl-N-methylacetamid |
| Vipy | : | N-Vinylpyrrolidon |
| AIBS | : | 2-Acrylamido-2-methylpropansulfonsäure |
| VSS-Na | : | Natriumvinylsulfonat |
| GBVP | : | Bis-vinylphosphonsäureglycolester |
| MBA | : | Methylenbisacrylamid |
| EBA | : | 1,2-Bis-acrylamido-ethan |
| BAME | : | Bis(acrylamidomethyl)ether |
| BAE | : | Bis-acrylamidoessigsäure |
| APS | : | Ammonpersulfat |
| KPS | : | Kaliumpersulfat |
| ABN | : | Azo-bis-isobutyronitril |
| ABAH | : | 2,2-Azo-bis(2-amidinopropan)dihydrochlorid |

Beispiel 1

In einem Polymerisationskolben von 2 l Inhalt, ausgestattet mit Planschliffdeckel, Rührer, Thermometer und Gaseinleitungsrohr werden in 560 ml entionisiertem Wasser 25,6 g Ätznatron und dann 38,4 g Borsäure gelöst. Anschließend werden 150 g AM, 40 g Vipy, 10 g AIBS und 0,1 g GBVP Rohprodukt zugesetzt und unter Rühren und Einleiten von Stickstoff bei 20°C innerhalb von 30 Minuten gelöst. Nun gibt man 120 mg APS- gelöst in 8 ml Wasser - zu und läßt unter Stickstoff-Einleitung noch 3 Minuten bei erhöhter Drehzahl rühren. Die $N_2$-Einleitung wird beendet, Einleitungsrohr und Rührer werden hochgezogen. Nach einer Induktionszeit von ca. 20 Minuten setzt die Polymerisation ein, wobei die Innentemperatur auf ca. 80°C ansteigt und die Lösung in ein formstabiles Gel übergeht. Das Polymerisat wird anschließend noch 8 Stunden auf 90°C geheizt, dann in Stücke geschnitten, getrocknet und durch Mahlung auf die gewünschte Korngröße gebracht.

Das als Vernetzer im obigen Beispiel eingesetzte GBVP-Rohprodukt wurde wie folgt hergestellt:
950 g Arbusow - Umlagerungsgemisch von Trischlorethylphosphit das 50 % 2-Chlorethanphosphonsäure-bis-2-chlorethylester und 16 % 2-Chlorethanphophonsäure-2-chlorethylester-2-(bis-/2-chlorethoxy7-phosphono)-ethylester enthält, werden mit 2 g Soda vermengt. 300 g dieses Gemenges werden unter Stickstoffatmosphäre und unter Rühren auf 170 bis 150°C erhitzt. Es beginnt Abspaltung von 1,2-Dichlorethan, das abdestilliert. Nun wird der Rest des Gemenges innerhalb von 4,5 Stunden unter steter Abdestillation von 1,2-Dichlorethan eingetropft. 4 Stunden wird nachgerührt, dabei wird zum Schluß die Temperatur auf etwa 200°C gesteigert. Es verbleiben 484 g GBVP-Rohprodukt, von dem 0,1 g,wie oben beschrieben, eingesetzt werden.

Beispiel 2

In einem Polymerisationskolben von 2 l Inhalt, ausgestattet mit Rührer, Rückflußkühler, Tropftrichter, Gaseinleitungsrohr und elektrisch beheiztem Wasserbad werden 1200 ml tert. Butanol vorgelegt und darin 60 g AIBS unter Rühren suspendiert, dann werden 6,6 l $NH_3$-Gas eingeleitet und anschließend 130 g Acrylamid, 10 g ViMA und 0,4 g MBA zugegeben. Unter Einleiten von Stickstoff wird mit dem elektrischen Wasserbad das Reaktionsgemisch auf 50°C geheizt und 500 mg ABN zugesetzt. Nach einer Induktionszeit von ca. 2 Stunden setzt die Polymerisation ein, die Reaktionstemperatur steigt bis auf ca. 70°C an und das Polymerisat fällt aus. Es wird noch 2 Stunden bei 80°C nachgeheizt, wobei eine dickflüssige Suspension entsteht. Das Polymere kann durch Absaugen und Trocknen unter Vakuum bei 50°C isoliert werden. Es kann jedoch auch das Lösungsmittel direkt unter vermindertem Druck aus dem Reaktionsgemisch abdestilliert werden, wodurch das Polymere in Form eines weißen Pulvers erhalten wird.

Beispiel 3

In einem Emulgierkolben von 2 l Inhalt mit gut wirkendem Rührer, Gaseinleitungsrohr und Bodenventil, der über dem Polymerisationkolben angeordnet ist, wird unter Stickstoff eine Emulsion folgender Zusammensetzung hergestellt: 400 ml ®Exol D, 350 ml entionisiertes Wasser, 20 g ®Arkopal N 230, 135 g AM, 50 g VSS-Na, 15 g AIBS, 4 g NaOH und 0,2 g BAE. Im Polymerisationskolben, ausgestattet mit Rührer, Rückflußkühler, Tropftrichter, Gaseinleitungsrohr, elektrisch beheiztem Wasserbad und Einleitungsrohr vom Emulgierkolben werden ca. 20 % der Emulsion vorgelegt. Unter Einleitung von

Stickstoff und Rühren wird auf 60°C geheizt. Dann werden über den Tropftrichter 20 % einer Lösung von 500 mg ABAH (Fa. Otsuka Chemical Co. Ltd., Japan) in 50 ml entionisiertem Wasser zugesetzt. Die Polymerisation beginnt nach etwa 30 Minuten unter deutlichem Temperaturanstieg auf ca. 80°C. Die restliche Emulsion wird gleichmäßig zusammen mit der verbliebenen Katalysatorlösung in 2 Stunden zugetropft. Danach wird noch 1 Stunde bei 70°C nachgeheizt. Es resultiert eine Dispersion des Polymers im organischen Lösungsmittel , aus der das Produkt ähnlich wie im Beispiel 2 isoliert werden kann.

Das in obigem Beispiel eingesetzte organische Lösungsmittel ®Exol ist eine Erdölfraktion mit einem Siedebereich von 200°C bis 240°C.

® Arkopal ist ein Emulgator auf Basis von Nonylphenol-polyglycoläther.

Gemäß diesen Herstellungsbeispielen können auch die Copolymerisate der Tabelle 1 hergestellt werden.

**T a b e l l e 1**

| Nr. | Monomerenzusammensetzung (%)<br>$CH_2{=}CH{-}R^1$ | <br>$R^2$ O<br>$CH_2{=}CH{-}N{-}C{-}R^3$ | <br>AM bzw.<br>MAM | Vernetzer (in % der übrigen Monomeren) | Weitere Zusätze (in % der Monom.) | Base | Katalys. | lt. Beispiel |
|---|---|---|---|---|---|---|---|---|
| 4 | - | $R^2{=}R^3{=}-CH_3$(5) | AM (95) | BAME(0,1) | $K_2CO_3$(13) | - | ABAH | 3 |
| 5 | $R^1{=}-PO(OC_2H_5)_2$(5)<br>$CH_3$<br>$R^1{=}-CONH{-}C{-}CH_2{-}SO_3H$(5)<br>$CH_3$ | - | AM (90) | MBA(0,025) | $K_2CO_3$(13) | NaOH | KPS | 1 |
| 6 | $R^1{=}-PO(OC_2H_5)_2$(5)<br>$CH_3$<br>$R^1{=}-CONH{-}C{-}CH_2{-}SO_3H$(5)<br>$CH_3$ | - | AM (90) | GBVP(0,1) | $K_2CO_3$(13) | NaOH | ABN | 2 |
| 7 | $R^1{=}-PO(OC_2H_5)_2$(5)<br>$CH_3$<br>$R^1{=}-CONH{-}C{-}CH_2SO_3H$(5)<br>$CH_3$ | - | AM (90) | GBVP(0,05) | $K_2CO_3$(13) | NaOH | ABAH | 3 |

Tabelle 1

| Nr. | Monomerenzusammensetzung (%) $CH_2=CH-R^1$ | $CH_2=CH-N(R^2)-C(=O)-R^3$ | AM bzw. MAM | Vernetzer (in % der übrigen Monomeren) | Weitere Zusätze (in % der Monom.) | Base | Katalys. | lt. Beispiel |
|---|---|---|---|---|---|---|---|---|
| 8 | $R^1 = -PO(OC_2H_5)_2$ (5)<br>$R^1 = -CONH-C(CH_3)_2-CH_2SO_3H$ (5) | - | AM (90) | GBVP (0,025) | $K_2CO_3$ (13) | NaOH | APS | 1 |
| 9 | $R^1 = -CONH-C(CH_3)_2-CH_2SO_3H$ (20) | $R^2 = R^3 = -CH_3$ (10) | AM (70) | MBA (0,05) | Na-Borat (4,3) | NaOH | APS | 1 |
| 10 | - | $R^2 = R^3 = -(CH_2)_3-$ (20) | AM (80) | GBVP (0,05) | Na-Borat (13,3) | NaOH | APS | 1 |
| 11 | $R^1 = -CONH-C(CH_3)_2-CH_2SO_3H$ (25) | $R^2 = R^3 = -CH_3$ (5) | AM (70) | GBVP (0,1) | - | NaOH | ABAH | 3 |
| 12 | - | $R^2 = R^3 = -CH_3$ (10) | AM (90) | MBA (0,05) | $K_2CO_3$ (13) | - | APS | 1 |
| 13 | - | $R^2 = R^3 = H$ (5) | AM (95) | MBA (0,2) | $K_2CO_3$ (13) | - | ABN | 2 |
| 14 | $R^1 = -CONH-C(CH_3)_2-CH_2SO_3H$ (20) | $R^2 = R^3 = -CH_3$ (10) | MAM (70) | EBA (0,05) | Na-Borat (4,3) | NaOH | APS | 1 |

Wie bereits oben gesagt sind die erfindungsgemäßen vernetzten Copolymerisate hervorragend geeignet als saugfähige Substanzen (sog. super absorbents ) in Hygiene-Artikeln, z.B. in Windeln, Damenbinden, Tampons, Papiertaschentüchern, usw.. Sie zeichnen sich insbesondere durch hohes Saugvermögen für physiologische und elektrolythaltige Flüssigkeiten wie Blut, Harn, Schweiß usw. aus.

Die Ausprüfung der erfindungsgemäßen Copolymeren im Hinblick auf ihre flüssigkeitsabsorbierende Wirkung in Hygienartikeln erfolgt unter anderem nach einer Schleudermethode mit Hilfe von Saugkörpern in Form von Monatsbinden.

Die Herstellung der Prüfkörper erfolgt, indem zunächst von käuflichen Binden die Umhüllung entfernt und das Flockenkissen gekürzt wird, daß es ein Gewicht von 5,0 g aufweist. Sodann wird das Flockenkissen mit einer Breite von ca. 6 cm und einer Länge von ca. 14 cm in der Dicke vorsichtig halbiert, und beide Hälften werden auseinandergeklappt. Anschließend werden 0,5 g Wirksubstanz(WS) des zu untersuchenden Copolymers in Granulatform auf eine Hälfte aufgebracht. Dazu verwendet man eine Schablone, so daß das Polymer auf einer Fläche von 4 x 8 cm zu liegen kommt. Sodann klappt man die andere Hälfte des Kissens zurück und wickelt den Bindenkörper neu ein mit einem siegelfähigen Vlies der Abmessungen 20 x 20 cm, verschweißt die offenen Enden durch Einwirkung von Wärmeenergie und schneidet das überstehende Vlies ab. Auf diese Weise erhält man Prüfkörper, die sowohl hinsichtlich ihrer Form als auch ihrer Zusammensetzung einer verkürzten Binde entsprechen. Auf die gleiche Weise werden Prüfkörper ohne Copolymer-Zusatz hergestellt. Die Prüfkörper werden anschließend gewogen und sodann 30 Minuten bzw. 3 Stunden in einer mit Prüfflüssigkeit gefüllten Schale so eingetaucht, daß sie sich ohne Behinderung vollsaugen können.

Als Prüflösung werden eine Blutersatzflüssigkeit (BE), bestehend aus 1%iger Kochsalzlösung, der noch Glyzerin, Natriumbicarbonat und etwas Hydroxyethylcellulose einer mittleren Viskosität von 20 mPa.s zugegeben wurden, verwendet, oder

eine Harnersatzflüssigkeit (HE); bei dieser handelt es sich ebenfalls um eine 1%ige Kochsalzlösung, die als weitere Bestandteile Harnstoff, Ammoniumphosphat (sek.), Kaliumsulfat und Zitronensäure enthält.

Nach der vorgegebenen Eintauchzeit werden die Prüfkörper durch kurzzeitiges Abschleudern von der nicht gebundenen Flüssigkeit befreit. Hierzu benutzt man eine marktgängige Wäscheschleuder mit einem Trommeldurchmesser von 23 cm und einer Umdrehungszahl von 1400 U/Min. Die Prüfkörper legt man an die Innenwand der Trommel und schleudert sie 20 Sekunden lang mit maximal 250-facher Erdbeschleunigung. (Die Zeit ist gerechnet vom Einschalten der Schleuder bis zum Abschalten und beinhaltet die Anlaufzeit bis zur Erreichung der vollen Tourenzahl). Anschließend werden die Prüfkörper wieder gewogen.

Zur besseren Vergleichbarkeit errechnet man das Rückhaltevermögen von einem Gramm Polymerisat wie folgt:

Rückhaltevermögen BE (HE) = (Gewicht des abgeschleuderten Saugkörpers mit Polymer - Gewicht des abgeschleuderten Saugkörpers ohne Polymer) x 2.

Das Ergebnis wird somit angegeben in (g Blutersatz/g WS-Polymer) oder ( g Harnersatz/g WS-Polymer).

In der folgenden Tabelle 2 sind die Ergebnisse der Ausprüfungen im Hinblick auf Rückhaltevermögen von Blutersatz und Harnersatzlösung aufgeführt.

Zum Vergleich zu den 10 erfindungsgemäßen Copolymerisaten 1 bis 10 ist ein handelsübliches hydrophiles synthetisches Polymeres auf der Basis von Polyacrylat eingesetzt worden.

T a b e l l e   2

| Eingesetztes Copolymerisat aus Herstellungsbeispiel Nr. | Rückhaltvermögen Blutersatzflüssigkeit (g BE/g WS Polymer) nach Eintauchzeiten von | | Rückhaltevermögen Harnersatzflüssigkeit (g HE/g WS Polymer) nach Eintauchzeiten von | |
|---|---|---|---|---|
| | 30 Minuten | 3 Stunden | 30 Minuten | 3 Stunden |
| 1 | 25,2 | 42,0 | 30,5 | 40,8 |
| 2 | 30,1 | 41,3 | 26,2 | 38,4 |
| 3 | 26,5 | 35,0 | 28,9 | 41,5 |
| 4 | 34,1 | 43,2 | 25,0 | 30,1 |
| 5 | 34,9 | 48,7 | 39,5 | 49,7 |
| 6 | 21,6 | 40,9 | 26,1 | 58,0 |
| 7 | 30,1 | 39,5 | 26,9 | 54,0 |
| 8 | 27,7 | 41,5 | 24,3 | 44,5 |
| 9 | 22,9 | 39,4 | 39,5 | 40,8 |
| 10 | 28,8 | 52,8 | 33,5 | 76,9 |
| Vergleich | 23,7 | 29,8 | 26,1 | 36,5 |

Ähnlich vorteilhafte Effekte werden mit den übrigen in Tabelle 1 angegebenen erfindungsgemäßen vernetzten Copolymerisaten erzielt.

## Patentansprüche

1. Wasserquellbare vernetzte Copolymerisate die in den Grundkettenin statistischer Verteilung zu

0 bis 60 Gew.% aus Resten der Formel I

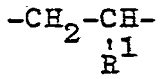
(I)

0 bis 40 Gew.% aus Resten der Formel II

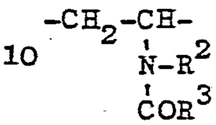
(II)

und 10 bis 95 Gew.% aus Resten der Formel III

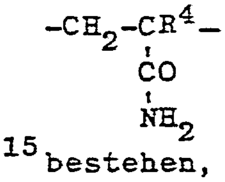
(III)

bestehen, wobei die Summe der Anteile der Reste der Formeln I und II 5 bis 90 Gew.% beträgt, und die zusätzlich 0,001 bis 2 Gew.%, bezogen auf das Gesamtgewicht der Grundkettenbestandteile, vernetzende Brückenglieder der Formeln IV und/oder V enthalten,

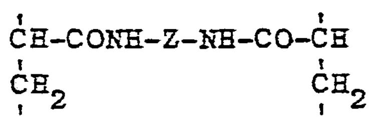
(IV)

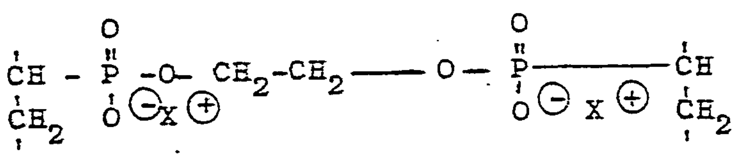
(V)

worin

$R^1$ einen der Reste der Formeln

$-CONH-C(CH_3)_2-CH_2-SO_3^{\ominus} X^{\oplus}$,

$-CO-NH-C(CH_3)_2-CH_2-PO_3^{\ominus\ominus} X_2^{\oplus}$

$-SO_3^{\ominus} X^{\oplus}$, $-PO_3^{\ominus\ominus} X_2^{\oplus}$ oder $-\overset{O}{\overset{\|}{P}}(OR^5)_2$ bedeutet,

$R^2$ und $R^3$ gleich oder verschieden sind und für Wasserstoff, Methyl, Ethyl oder gemeinsam für Trimethylen stehen, $R^4$ Wasserstoff oder Methyl, $R^5$ Alkyl mit 1 bis 4 C-Atomen, $X^{\oplus}$ ein Kation und Z eine der Gruppen $-CH_2-$, $-CH_2CH_2-$,

$COO^{\ominus}X^{\oplus}$
$-\overset{|}{CH}-$ oder $-CH_2-O-CH_2-$ ist sowie deren

Partialhydrolysate, in denen ein Anteil von bis zu 60 % der ursprünglich vorhandenen Gruppen der Formel III zu Gruppen der Formel VI

$$-CH_2-\underset{COO^{\ominus}X^{\oplus}}{\underset{|}{CH}}- \qquad (VI)$$

hydrolysiert wurden, wobei die Produkte insgesamt 10 bis 90 Gew.% anionische Reste aufweisende Gruppen der Formeln I und VI enthalten.

2. Wasserquellbare, vernetzte Copolymerisate gemäß Anspruch 1, dadurch gekennzeichnet, daß die Grundketten in statistischer Verteilung zu
0 bis 50 Gew.% aus Resten der Formel I
0 bis 30 Gew.% aus Resten der Formel II und zu
20 bis 95 Gew.% aus Resten der Formel III
bestehen.

3. Wasserquellbare, vernetzte Copolymerisate gemäß den Ansprüchen 1 und 2, dadurch gekennzeichnet, daß sie, bezogen auf das Gesamtgewicht der Grundkettenbestandteile 0,02 bis 0,5 Gew.% vernetzende Brückenglieder der Formeln IV und/oder V enthalten.

4. Wasserquellbare, vernetzte Copolymerisate gemäß den Ansprüchen 1 bis 3, dadurch gekennzeichnet, daß in den darin enthaltenen Resten der Formel I
$R^1$ eine Gruppe der Formel

$-CO-NH-C(CH_3)_2-CH_2-SO_3^{\ominus} X^{\oplus}$,

$-PO_3^{\ominus\ominus} X_2^{\oplus}$ oder $-\overset{O}{\overset{\|}{P}}(OC_2H_5)_2$ ist.

5. Wasserquellbare, vernetzte Copolymerisate gemäß den Ansprüchen 1 bis 4, dadurch gekennzeichnet, daß sie insgesamt 20 bis 80 Gew.% anionische Reste aufweisende Gruppen der Formeln I und VI enthalten.

6. Wasserquellbare, vernetzte Copolymerisate gemäß den Ansprüchen 1 bis 5, dadurch gekennzeichnet, daß sie zusätzlich 2,5 bis 35 Gew.% Borat Anionen, berechnet als $H_3BO_3$ und bezogen auf das Gewicht des nicht hydrolysierten Copolymerisats, enthalten.

7. Verfahren zur Herstellung von wasserquellbaren, vernetzten Copolymerisaten der in Anspruch 1 angegebenen Zusammensetzung, dadurch gekennzeichnet, daß man
0 bis 60 Gewichtsteile einer Vinylverbindung der Formel Ia

$$CH_2 = CH-R^1 \qquad (Ia),$$

sofern sie Sulfo- oder Phosphonsäuregruppen enthält, neutralisiert, und mit
0 bis 40 Gewichtsteilen einer Vinylverbindung der Formel IIa

$$CH_2 = CH-\overset{R^2}{\overset{|}{N}}-CO-R^3 \qquad (IIa),$$

10 - 95 Gewichtsteilen Acrylamid und/oder Methacrylamid sowie m Gewichtsteilen von Bis-vinylverbindungen der Formeln IVa + Va

$$CH_2=CH-CONH-Z-NH-CO-CH=CH_2 \qquad (IVa)$$

$$CH_2=CH-\underset{O^{\ominus}X^{\oplus}}{\underset{|}{\overset{O}{\overset{\|}{P}}}}-O-CH_2-CH_2-O-\underset{O^{\ominus}X^{\oplus}}{\underset{|}{\overset{O}{\overset{\|}{P}}}}-CH=CH_2 \qquad (Va)$$

in an sich bekannter Weise copolymerisiert, wobei die Gesamtmenge der Vinylverbindungen der Formeln Ia und IIa 5 bis 95 Gewichtsteile und die Gesamtmenge der Vinylverbindungen der Formeln Ia, IIa und Acrylamid 100 Gewichtsteile beträgt, m eine Zahl von 0,001 bis 2 ist und $R^1$, $R^2$, $R^3$, $X^{\oplus}$, und Z die in Anspruch 1 genannten Bedeutungen haben,

und daß man gegebenenfalls während oder nach der Copolymerisation bis zu 60% der in den vernetzten Copolymerisaten enthaltenen Acrylamidbausteine zu Gruppen der Formel VI

$$-CH_2-\underset{\displaystyle COO^{\ominus}\ X^{\oplus}}{\underset{|}{CH}}- \qquad (VI)$$

hydrolisiert.

8. Verfahren gemäß Anspruch 7, dadurch gekennzeichnet, daß man 0 bis 50 Gew.Teile Vinylverbindungen der Formel Ia, 0 bis 30 Gew.Teile Vinylverbindungen der Formel IIa und 20 bis 95 Gew. Teile Acrylamid einsetzt.

9. Verfahren gemäß den Ansprüchen 7 und 8, dadurch gekennzeichnet, daß man 0,02 bis 0,5 Gew.-Teile von Bis-vinylverbindungen der Formeln IVa und/oder Va einsetzt.

10. Verwendung der wasserquellbaren vernetzten Copolymerisate des Anspruchs 1 als Absorbentien für wäßrige Flüssigkeiten.

0068159

Europäisches Patentamt

# EUROPÄISCHER RECHERCHENBERICHT

Nummer der Anmeldung

EP 82 10 4746

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 044 508 (CASELLA AG)<br>* Anspruch 1 *<br>--- | 1-8 | C 08 F 220/56<br>A 61 L 15/00 //<br>(C 08 F 220/56<br>C 08 F 226/02<br>C 08 F 220/58<br>C 08 F 230/02 ) |
| A | EP-A-0 023 712 (CASELLA AG)<br>* Anspruch 1 *<br>--- | 1-8 | |
| A | DE-B-2 444 108 (HOECHST)<br>* Anspruch 1 *<br>--- | 1-8 | |
| A | FR-A-2 149 714 (TAMPAX)<br>* Anspruch 1 *<br>----- | 9,10 | |
| | | | RECHERCHIERTE SACHGEBIETE (Int. Cl. 3) |
| | | | C 08 F<br>A 61 L |

Der vorliegende Recherchenbericht wurde für alle Patentansprüche erstellt.

| Recherchenort | Abschlußdatum der Recherche | Prüfer |
|---|---|---|
| DEN HAAG | 17-09-1982 | CAUWENBERG C.L.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82